# EUROPÄISCHE PATENTSCHRIFT

(11) **EP 0 951 266 B1**
(45) Veröffentlichungstag und Bekanntmachung des Hinweises auf die Patenterteilung: **27.03.2002**
(21) Anmeldenummer: 97952873.4
(22) Anmeldetag: 04.12.1997
(51) Int. Cl.: A61F 13/58

(54) **HYGIENEARTIKEL ZUM EINMALIGEN GEBRAUCH MIT KOMBINIERTEM MECHANISCHEM UND KLEBENDEM VERSCHLUSSSYSTEM**
SINGLE USE HYGIENE ARTICLE WITH COMBINED MECHANIC AND ADHESIVE CLOSING SYSTEM
ARTICLE D'HYGIENE A USAGE UNIQUE AVEC SYSTEME DE FERMETURE MECANIQUE ET ADHESIF

(30) Priorität: 23.12.1996 DE 19654052
(43) Veröffentlichungstag der Anmeldung: 27.10.1999
(73) Patentinhaber: Paul Hartmann Aktiengesellschaft, 89522 Heidenheim (DE)
(72) Erfinder: MALOWANIEC, Krzysztof, D-89522 Heidenheim (DE)
(74) Vertreter: Dreiss, Fuhlendorf, Steimle & Becker
(86) Internationale Anmeldenummer: EP9706773
(87) Internationale Veröffentlichungsnummer: WO9827922

(56) Entgegenhaltungen:
- EP-A- 0 393 953
- EP-A- 0 418 954
- WO-A-96/25905
- DE-U- 29 711 430

## Beschreibung

Die Erfindung betrifft einen Hygieneartikel zum einmaligen Gebrauch gemäß dem Oberbegriff des Anspruchs 1.

Bekannte absorbierende Wegwerfartikel, wie Windeln, Inkontinenzprodukte oder dergleichen, weisen einen Absorptionskörper und ein Vorder- und Rückteil auf. Beim Anlegen des Artikels an einen Träger werden Vorder- und Rückteil über Verschlussmittel miteinander verbunden. Die Verschlussmittel, die in der Regel aus an Seitenrändern des Rückteils angeordneten Klebestreifen bestehen, sind zum Abnehmen des verschmutzten Artikels vom Vorderteil wieder lösbar. Der gebrauchte Wegwerfartikel kann dann zu dessen Entsorgung zu einem kleinen Päckchen gefaltet oder zusammengerollt werden. Wenn die Klebebänder von außen auf den zusammengerollten Artikel befestigt werden, besteht nicht die Gefahr, daß beim Wegwerfen des Artikels die vom absorbierenden Artikel aufgenommenen Körperausscheidungen aus dem zusammengerollten Artikel entweichen.

Wenn die Verschlussmittel als Klebebänder ausgebildet sind, sind diese an einem beliebigen Teil der Windel festlegbar, also insbesondere auch auf dem zu einem kleinen Päckchen zusammengerollten Artikel. Klebebänder haben jedoch den großen Nachteil, dass sie durch Öl, Creme, Puder oder dergleichen verschmutzen und damit nicht mehr haften, so dass die Windel nicht mehr geschlossen werden kann. Deshalb geht man dazu über, die Klebebänder durch mechanische Befestigungsmittel, beispielsweise Klettverschlüsse, zu ersetzen. Die Klebebänder werden ersetzt durch Verschlusslaschen, die erste mechanische Verschlusselemente, beispielsweise Häkchen, aufweisen, die zum Schliessen des an einen Träger angelegten Artikels mit zweiten mechanischen Verschlusselementen, beispielsweise am Vorderteil angeordnete Ösen, verbunden werden. Dann besteht jedoch die beschriebene Möglichkeit, den Wegwerfartikel im zusammengerollten Zustand zu halten, nicht mehr, denn die den einen Teil des Klettverschlusses aufweisenden Verschlußlaschen sind nicht mehr an der zusammengerollten Windel festlegbar, da die entsprechen den komplementären Verschlussteile eingerollt sind.

Zur Lösung dieses Problems wird gemäß der EP 0 321 232 ein absorbierender Wegwerfartikel mit Verschlussmitteln vorgeschlagen, bei dem die Verschlussmittel mechanische Verschlusselemente und daneben mit Klebstoff versehene Bereiche aufweisen, so dass die Verschlussmittel trotz der mechanischen Verschlussmittel auch an dem zusammengerollten Artikel festlegbar sind. Nachteilig an diesen bekannten Verschlußmitteln ist, daß sie entsprechend groß, insbesondere lang, ausgelegt sein müssen, damit sowohl die mechanischen Verschlusselemente und die mit Klebstoff versehenen Bereiche Platz finden. Der entsprechende Wegwerfartikel ist daher kostenungünstig.

Aus der EP 0 418 954 A2 ist ein Befestigungssystem insbesondere für Hygieneartikel mit sowohl mechanisch als auch klebend zusammenwirkenden komplementär ineinander greifbaren Oberflächenstrukturen bekannt.

Die WO 96/25905 lehrt, bei einem Klettverschlussbefestigungssystem sowohl die hakenbildende Komponente als auch die schlaufenbildende Komponente zusätzlich mit einem Kohäsionskleber zu versehen, um die erwünschten Hafteigenschaften (Scherkräfte und Abziehkräfte) zwischen den Klettverschlusskomponenten einstellen zu können.

Aus der EP 0 393 953 ist ein Klebstoffauftrag bekannt, um beispielsweise eine Monatsbinde an einem Kleidungsstück festzulegen. Damit die Binde an dem textilen Material des Kleidungsstücks besser haftet, sind auf der die Klebstoffschicht aufweisenden Seite der Binde Vorsprünge vorgesehen, die in das textile Material eindrücken und so die Binde rutschsicherer festlegen. Für eine Wegwerfwindel, die in der Regel Kunststofffolien und keine textilen Materialien aufweist, ist eine Klebstoffschicht mit Vorsprüngen nicht geeignet, da die Vorsprünge nicht in die Folien eindringen können, so dass eine derartige Klebstoffschicht nicht an einer Wegwerfwindel festlegbar ist.

Ausgehend von diesem Stand der Technik ist es Aufgabe der Erfindung, einen verbesserten Hygieneartikel zum einmaligen Gebrauch bereitzustellen, der verbesserte, einfach aufgebaute und zuverlässig schließende Verschlusslaschen mit mechanischen Verschlusselementen und mit wenigstens einem klebend ausgebildeten Bereich aufweist. Der Artikel soll sicher entsorgbar und ein ungewolltes Öffnen des zusammengerollten oder -gefalteten Artikels verhinderbar sein.

Diese Aufgabe wird gelöst durch einen gattungsgemässen Hygieneartikel mit den kennzeichnenden Merkmalen des Anspruchs 1.

Wenn wenigstens ein Teil der mechanischen Verschlusselemente der ersten Art an ihrem von der Verschlusslasche abgewandten Ende jeweils einen Klebstoffauftrag aufweisen und diese Verschlusselemente den klebend ausgebildeten Bereich bilden, haben diese Verschlusselemente sowohl eine mechanische Funktion beim Schließen des Artikels als auch zugleich eine klebende Funktion beim Festlegen der Verschlusslaschen an der zur Entsorgung zusammengerollten oder -gefalteten Windel an einem anderen Teil außerhalb der zweiten mechanisch wirkenden Verschlusselemente. Daraus ergibt sich der weitere Vorteil, dass die Windel stets sicher beim Anlegen an einen Träger verschließbar ist, denn wenn beispielsweise eine Verschlusslasche beim Schließen nicht exakt auf der Windel positioniert wird, so dass nur ein Teil der mechanischen Verschlusselemente der ersten Art mit den Verschlusselementen der zweiten Art in Eingriff gelangen, sind die Verschlusselemente der ersten Art dennoch aufgrund des erfindungsgemässen Klebstoffauftrags an der Windel festgelegt. Dieser Vorteil kann durch die bekannten und separat von den mechanischen Verschlusselementen der ersten Art angeordneten mit Klebstoff versehenen Bereiche nicht erreicht werden.

Die Verschlusslaschen des erfindungsgemäßen Artikels können aufgrund der Doppelfunktion der ersten Verschlusselemente kürzer ausgebildet werden, wodurch sich eine Materialersparnis ergibt. Der klebend ausgebildete Bereich wird weniger schnell verschmutzen als die bekannten und von den mechanischen Verschlusselementen separat angeordneten mit Klebstoff versehenen Bereiche, da die Verschmutzungen, beispielsweise Öl, von dem einzelnen Klebstoffauftrag eines Verschlusselements in die Zwischenräume zwischen den Verschlusselementen "ablaufen" kann. In den Zwischenräume stört die Verschmutzung jedoch nicht, so daß der erfindungsgemäße Artikel unempfindlicher gegenüber Verschmutzungen der Verschlusslaschen ist.
Bevorzugt weisen im wesentlichen alle Verschlusselemente der ersten Art einen Klebstoffauftrag auf, um eine maximale Klebekraft zu erhalten. In einer einfachen Ausgestaltung der Erfindung sind die Verschlusselemente der ersten Art wie bei bekannten Klettverschlüsse als Häkchen ausgebildet. Bevorzugt weisen die Verschlusselemente der ersten Art jedoch eine pilzartige Form mit Stil und Kopf auf, denn dann kann eine grössere Menge Klebstoff auf das einzelne Verschlusselement aufgebracht werden.

Um die auf ein Verschlusselement aufgebrachte Klebstoffmenge weiter erhöhen zu können ist der Kopf der Verschlusselemente der ersten Art abgeflacht und das Verschlusselement weist im Querschnitt bevorzugt eine T-Form auf.

In einer Ausgestaltung der Erfindung gemäß Anspruch 7 ist zwischen den Verschlusselementen der ersten Art wenigstens bereichsweise ein weiterer Klebstoffauftrag vorgesehen, wodurch die Klebekraft weiter erhöht wird, wenn die Verschlusslasche stark auf das Material gepresst wird, auf der sie festgelegt werden soll, so daß die Verschlusselemente der ersten Art entweder verbogen werden, oder in das Material eindringen.

Bevorzugt ändert sich die Dicke des weiteren Klebstoffauftrags in Längsrichtung der Verschlusslasche, um Bereiche mit unterschiedlich großer Klebekraft zu schaffen.

Bevorzugt nimmt die Dicke des weiteren Klebstoffauftrags in Richtung des freien Laschenendes hin ab, so daß beim Öffnen des an einen Träger angelegten Artikels, das weniger große Klebekraft aufweisende Verschlusslaschenende leichter zu lösen ist.
Im folgenden wird die Erfindung anhand von Ausführungsbeispielen unter Bezugnahme auf die Zeichnung im einzelnen erläutert. In der Zeichnung zeigen:
- Fig. 1:: einen erfindungsgemäßen Hygieneartikel im flachgelegten Zustand;
- Fig. 2 und 3:: Ausführungsbeispiele von Verschlusslaschen des Hygieneartikels im Querschnitt.

Ein in der Zeichnung dargestellter erfindungsgemäßer Hygieneartikel 10 zum einmaligen Gebrauch kann eine Wegwerfwindel, Inkontinenzprodukt oder dgl. sein. Im folgenden wird der Hygieneartikel als Windel bezeichnet. In den Figuren der Zeichnung sind gleiche Teile mit gleichen Bezugsziffern bezeichnet.

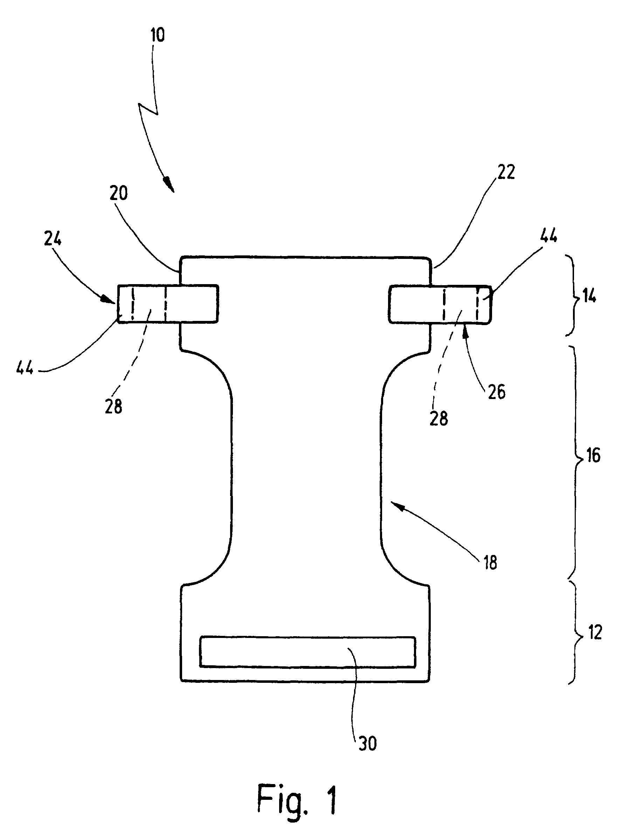

In Fig. 1 ist die Windel 10 im flachgelegten Zustand und von der einem Träger abgewandten Seite, also der Außenseite, her gesehen dargestellt. Die Windel 10 weist ein Vorderteil 12, ein Rückteil 14 und einen dazwischen angeordneten Schrittbereich 16 auf. In bekannter und nicht näher dargestellter Weise ist die Windel 10 bevorzugt aus einer flüssigkeitsundurchlässigen Rückschicht, einem flüssigkeitsdurchlässigem Abdeckvlies und dazwischen angeordnetem Saugkörper, beispielsweise aus Zelluloseflocken und ggf. superabsorbierenden Stoffen, gebildet. Zur besseren Abdichtung der Windel 10 an den Beinen eines Trägers können im Bereich von Beinausschnitten 18 im Schrittbereich 16 elastische Elemente vorgesehen sein.

Das Vorderteil 12 und das Rückteil 14 weisen jeweils einen Taillenrand und Seitenränder 20 und 22 auf. Am vorderen und/oder hinteren Taillenrand kann ein elastisches Element vorgesehen sein, so dass der vordere und/oder hintere Taillenrandbereich" elastisch ausgebildet ist, um die Abdichtung der Windel 10 im Bereich der Taille gegenüber dem Körper eines Trägers zu verbessern.

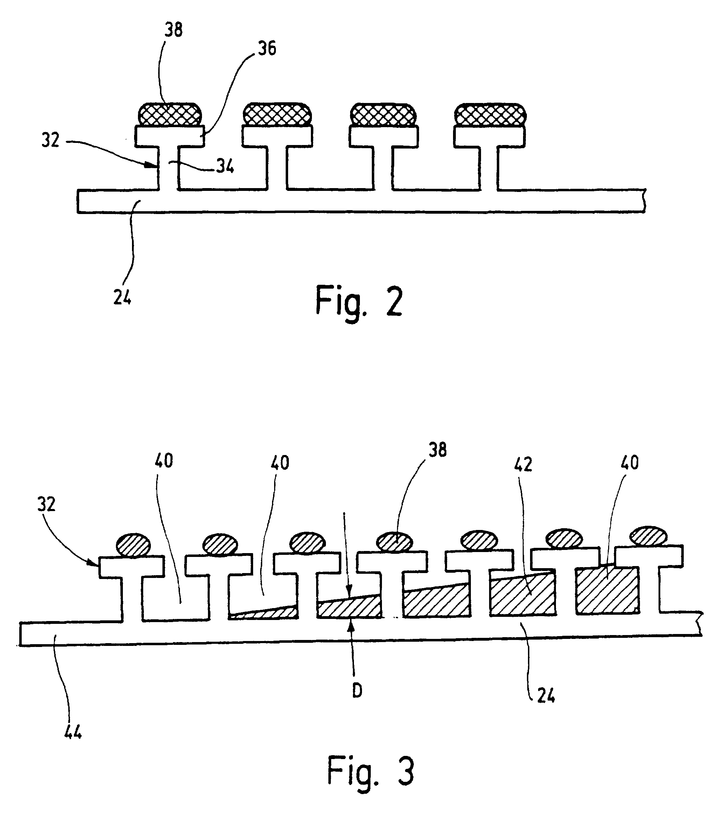

Weiter weist die Windel 10 an den Seitenrändern 20 und 22 je eine Verschlusslasche 24 und 26 auf, mittels derer das Rückteil 14 mit dem Vorderteil 12 über die Seitenränder verbindbar ist, um die an den Träger angelegte Windel schließen zu können. Dazu sind die in Fig. 2 und 3 näher dargestellten Verschlusslaschen 24 und 26 mit wenigstens einem Verschlussteil 30, beispielsweise ein Ösen aufweisender Klettflausch, in Eingriff bringbar.

In den Fig. 2 und 3 ist die Verschlusslasche 24 bzw. 26 vergrössert und im Querschnitt dargestellt. Die Verschlusslasche weist wenigstens auf einem Teilbereich 28 mechanische Verschlusselemente 32 einer ersten Art auf, die mit nicht näher dargestellten Verschlusselementen einer zweiten Art des Verschlussteils 30 mechanisch verbindbar sind. Die Verschlusselemente 32 der ersten Art können beispielsweise die Häkchen eines bekannten Klettverschlusses sein, die mit Ösen des Verschlussteils 30 zusammenwirken können.

Bevorzugt weisen die Verschlusselemente 32 der ersten Art jedoch eine pilzartige Form mit einem Stil 34 und einem Kopf 36 auf, wobei der Kopf 36 bevorzugt abgeflacht ist und die Verschlusselemente 32 der ersten Art im in Fig. 2 und 3 dargestellten Querschnitt im wesentlichen eine T-Form aufweisen. Diese T-förmigen Verschlusselemente 32 können wie die genannten Häkchen mit den Ösen des Verschlussteils 30 mechanisch zusammenwirken.

Auf ihrem von der Verschlusslasche 24 abgewandten Ende, also in dem, dargestellten Ausführungsbeispiel auf ihrem Kopf 36, weisen wenigstens ein Teil der Verschlusselemente 32 der ersten Art jeweils einen Klebstoffauftrag 38 auf. Bevorzugt weisen jedoch im wesentlichen alle Verschlusselemente 32 den Klebstoffauftrag 38 auf. Dabei kann der Klebstoffauftrag 38 den ganzen Kopf 36 oder nur einen Teil des Kopfes 36 (Fig. 3) bedecken. Es handelt sich um einen Adhäsionskleber, so dass die Verschlusslaschen 24 und 26 mit ihren Verschlusselementen (32) der ersten Art auch außerhalb des Verschlussteils 30 an einem anderen Teil des Artikels festgelegt werden können, um den Artikel in zusammengefalteter oder zusammengerollter Konfiguration halten und sicher entsorgen zu können. Der Verschlussteil 30, der die Verschlusselemente der zweiten Art aufweist, ist klebstofffrei und bildet mit den Verschlußelementen der Verschlusslaschen 24 und 26 lediglich eine mechanische Halteverbindung aus.

In einem weiteren in Fig. 3 dargestellten Ausführungsbeispiel ist in Zwischenräumen 40 zwischen den Verschlusselementen 32 der ersten Art wenigstens bereichsweise ein weiterer Klebstoffauftrag 42 vorgesehen. Bevorzugt ändert sich die Dicke D dieses weiteren Klebstoffauftrags 42 in Längsrichtung der Verschlusslasche 24 bzw. 26. Dabei ist es vorteilhaft, wenn die Dicke D in Richtung eines freien Laschenendes 44 hin kontinuierlich, also graduell, abnimmt. Gegebenenfalls weisen die dem freien Laschenende 44 benachbarten Zwischenräume 40' keinen Klebstoff auf und die dem an der Windel befestigten Laschenende benachbarten Zwischenräume 40' sind im wesentlichen vollständig mit Klebstoff gefüllt.

Das freie Laschenende 44 weist bevorzugt weder mechanische Verschlusselemente noch einen Klebstoffauftrag auf und kann daher stets zum Lösen der festgelegten Verschlusslasche ergriffen werden.

## Patentansprüche

1. Hygieneartikel zum einmaligen Gebrauch, insbesondere Wegwerfwindel oder Inkontinenzprodukt, mit einem Vorderteil (12), einem Rückteil (14) und dazwischen liegendem Schrittbereich (16), mit an Seitenrändern (20 und 22) des Rückteils (14) angeordneten Verschlusslaschen (24 und 26) zum Verbinden des Rückteils (14) mit dem Vorderteil (12) zum Schliessen des an einen Träger angelegten Artikels (10), wobei die Verschlusslaschen (24 und 26) erste mechanische Verschlusselemente (32) aufweisen, die mit zweiten, am Vorderteil (12) angeordneten, mechanischen Verschlusselementen beim Schliessen des Artikels zusammenwirken und wobei die Verschlusslaschen (24 und 26) jeweils wenigstens einen klebend ausgebildeten Bereich aufweisen, um die Verschlusslaschen (24, 26) auch an einem anderen Teil des Artikels (10) festlegen zu können, **dadurch gekennzeichnet, dass** wenigstens ein Teil der mechanischen Verschlusselemente (32) der ersten Art an ihrem von der Verschlusslasche (24, 26) abgewandten Ende jeweils einen Klebstoffauftrag (38) aufweisen und diese Verschlusselemente (32) zugleich den klebend ausgebildeten Bereich bilden und damit sowohl eine mechanische Funktion beim Schliessen des Artikels als auch zugleich eine klebende Funktion beim Festlegen der Verschlusslaschen an einem anderen Teil des gefalteten oder zusammengerollten Artikels zum Entsorgen des Artikels haben.

2. Hygieneartikel nach Anspruch 1, **dadurch gekennzeichnet, dass** im wesentlichen alle Verschlusselemente (32) der ersten Art einen Klebstoffauftrag (38) aufweisen.

3. Hygieneartikel nach Anspruch 1 oder 2, **dadurch gekennzeichnet, dass** die Verschlusselemente (32) der ersten Art als Häkchen ausgebildet sind.

4. Hygieneartikel nach Anspruch 1 oder 2, **dadurch gekennzeichnet, dass** die Verschlusselemente (32) der ersten Art eine pilzartige Form mit Stil (34) und Kopf (36) aufweisen.

5. Hygieneartikel nach Anspruch 3, **dadurch gekennzeichnet, dass** der Kopf (36) der Verschlusselemente (32) der ersten Art abgeflacht ist.

6. Hygieneartikel nach Anspruch 5, **dadurch gekennzeichnet, dass** die Verschlusselemente (32) der ersten Art im Querschnitt eine T-Form aufweisen.

7. Hygieneartikel nach einem der vorhergehenden Ansprüche, **dadurch gekennzeichnet, dass** zwischen den Verschlusselementen (32) der ersten Art wenigstens bereichsweise ein weiterer Klebstoffauftrag (42) vorgesehen ist.

8. Hygieneartikel nach Anspruch 7, **dadurch gekennzeichnet, dass** die Dicke (D) des weiteren Klebstoffauftrags (42) in Längsrichtung der Verschlusslasche (24, 26) sich ändert.

9. Hygieneartikel nach Anspruch 8, **dadurch gekennzeichnet, dass** die Dicke (D) des weiteren Klebstoffauftrags (42) in Richtung des freien Laschenendes (44) hin kontinuierlich abnimmt.

## Claims

1. Single-use sanitary article, in particular disposable nappy or incontinence product, with a front part (12), a back part (14) and a crotch region (16) lying therebetween, with fastening tabs (24 and 26) arranged on lateral edges (20 and 22) of the back part (14) for connecting the back part (14) to the front part (12) to close the article (10) placed on a wearer, the fastening tabs (24 and 26) having first mechanical fastening elements (32) which co-operate with second mechanical fastening elements arranged on the front part (12) when the article is closed and the fastening tabs (24 and 26) each having at least one region which is designed to be adhesive so as also to be able to fasten the fastening tabs (24, 26) on another part of the article (10), **characterised in that** at least some of the mechanical fastening elements (32) of the first type have a respective application of adhesive (38) on their end remote from the fastening tab (24, 26) and these fastening elements (32) simultaneously form the region which is designed to be adhesive and therefore have both a mechanical function when the article is being closed and also simultaneously an adhesive function when the fastening tabs are being fastened to another part of the folded or rolled up article for the disposal of the article.

2. Sanitary article according to claim 1, **characterised in that** substantially all the fastening elements (32) of the first type have an application of adhesive (38).

3. Sanitary article according to claim 1 or 2, **characterised in that** the fastening elements (32) of the first type are designed as small hooks.

4. Sanitary article according to claim 1 or 2, **characterised in that** the fastening elements (32) of the first type have a mushroom-like shape with a stalk (34) and head (36).

5. Sanitary article according to claim 3, **characterised in that** the head (36) of the fastening elements (32) of the first type is flattened.

6. Sanitary article according to claim 5, **characterised in that** the fastening elements (32) of the first type have a T-shape in cross-section.

7. Sanitary article according to any one of the preceding claims, **characterised in that** a further application of adhesive (42) is provided at least in regions between the fastening elements (32) of the first type.

8. Sanitary article according to claim 7, **characterised in that** the thickness D of the further application of adhesive (42) changes in the longitudinal direction of the fastening tab (24, 26).

9. Sanitary article according to claim 8, **characterised in that** the thickness D of the further application of adhesive (42) reduces continuously in the direction of the free tab end (44).

## Revendications

1. Article d'hygiène à usage unique, notamment couche jetable ou produit d'incontinence comprenant une partie avant (12), une partie arrière (14) et l'entrejambe (16) entre ces deux parties, avec des languettes de fermeture (24 et 26) disposées sur les bords latéraux (20 et 22) de la partie arrière (14) pour relier la partie arrière (14) à la partie avant (12) afin de fermer l'article (10) positionné sur un utilisateur, les languettes de fermeture (24 et 26) comprenant un premier ensemble d'éléments de fermeture mécaniques (32) coopérant avec un deuxième ensemble d'éléments de fermeture mécaniques disposés sur la partie avant (12) lors de la fermeture de l'article, les languettes de fermeture (24 et 26) comprenant chacune au moins une zone adhésive afin de permettre de fixer les languettes de fermeture (24, 26) également sur une autre partie de l'article (10), **caractérisé en ce qu'**au moins une partie des éléments de fermeture mécaniques (32) du premier type comprennent, sur leur extrémité opposée à la languette de fermeture (24, 26), une couche de colle (38) et **en ce que** ces éléments de fermeture (32) constituent en même temps la zone adhésive de façon à pouvoir remplir à la fois une fonction mécanique lors de la fermeture de l'article et une fonction adhésive lors de la fixation des languettes de fermeture sur une autre partie de l'article plié ou enroulé en vue de son élimination.

2. Article d'hygiène selon la revendication 1, **caractérisé en ce que** sensiblement tous les éléments de fermeture (32) du premier type comprennent une couche d'adhésif (38).

3. Article d'hygiène selon la revendication 1 ou 2, **caractérisé en ce que** les éléments de fermeture (32) du premier type sont réalisés en forme de petits crochets.

4. Article d'hygiène selon la revendication 1 ou 2, **caractérisé en ce que** les éléments de fermeture (32) du premier type sont réalisés en forme de petits champignons comprenant une tige (34) et une tête (36).

5. Article d'hygiène selon la revendication 3, **caractérisé en ce que** la tête (36) des éléments de fermeture (32) du premier type est aplatie.

6. Article d'hygiène selon la revendication 5, **caractérisé en ce que** les éléments de fermeture (32) du premier type présentent une section en forme de "T".

7. Article d'hygiène selon l'une quelconque des revendications précédentes, **caractérisé en ce qu'**une couche d'adhésif supplémentaire (42) est appliquée au moins dans certaines zones entre les éléments de fermeture (32) du premier type.

8. Article d'hygiène selon la revendication 7, **caractérisé en ce que** l'épaisseur (D) de la couche d'adhésif supplémentaire (42) varie dans le sens longitudinal de la languette de fermeture (24, 26).

9. Article d'hygiène selon la revendication 8, **caractérisé en ce que** l'épaisseur (D) de la couche d'adhésif supplémentaire (42) diminue progressivement en direction de l'extrémité libre (44) de la languette.
